Europäisches Patentamt

European Patent Office

Office européen des brevets

(19)

(11) Veröffentlichungsnummer : **0 091 575 B1**

(12) **EUROPÄISCHE PATENTSCHRIFT**

(45) Veröffentlichungstag der Patentschrift :
21.01.87

(21) Anmeldenummer : 83102757.8

(22) Anmeldetag : 21.03.83

(51) Int. Cl.⁴ : **C 07 C 76/06**, C 07 C 79/36// C07C79/343

(54) Verfahren zur Herstellung von o-Nitrobenzaldehyd.

(30) Priorität : 01.04.82 DE 3212069

(43) Veröffentlichungstag der Anmeldung :
19.10.83 Patentblatt 83/42

(45) Bekanntmachung des Hinweises auf die Patenterteilung : 21.01.87 Patentblatt 87/04

(84) Benannte Vertragsstaaten :
AT BE CH DE FR GB IT LI LU NL SE

(56) Entgegenhaltungen :
DE-C- 116 124
Chemical Abstracts Band 69, Nr. 15, 7. Oktober 1968, Columbus, Ohio, USA M.M. BURSEY "Photochemical analogies in mass spectra. An ortho effect Seite 5477, Spalte 1, Abstract Nr. 58724e
Die Akte enthält technische Angaben, die nach dem Eingang der Anmeldung eingereicht wurden und die nicht in dieser Patentschrift enthalten sind.

(73) Patentinhaber : BAYER AG
Konzernverwaltung RP Patentabteilung
D-5090 Leverkusen 1 Bayerwerk (DE)

(72) Erfinder : Preiss, Michael, Dr.
Egenstrasse 21
D-5600 Wuppertal 1 (DE)
Erfinder : Gau, Wolfgang, Dr.
Am Eckbusch 39-56
D-5600 Wuppertal 1 (DE)
Erfinder : Behre, Horst, Dr.
Zur alten Linde 12
D-5068 Odenthal (DE)

**Beschreibung**

Die vorliegende Erfindung betrifft ein neues fortschrittliches Verfahren zur Herstellung von 2-Nitrobenzaldehyd, welches als Zwischenprodukt vielseitig verwendbar ist und insbesondere bei der Herstellung von pharmazeutisch wirksamen 4-(2-Nitrophenyl)-1,4-dihydropyridin-Derivaten verwendet werden kann (vgl. DT-PS-1 670 827).

2-Nitrobenzaldehyd war bisher nur schwer zugänglich, da die meisten klassischen Verfahren der Aldehydsynthese bei dieser Verbindung versagen. In der Deutschen Auslegeschrift 2 415 062 werden die Nachteile der literaturbekannten Verfahren ausführlich kommentiert. Das in dieser Auslegeschrift beschriebene Herstellungsverfahren aus 2-Nitrotoluol über die Zwischenstufen 2-Nitrophenylbrenztraubensäure und 2-Nitrobenzalchlorid liefert zwar den 2-Nitrobenzaldehyd in guten Ausbeuten, ist aber als technisches Verfahren immer noch aufwendig, bedingt durch die Art und die Anzahl der Reaktions- und Reinigungsschritte.

Als weiterer Herstellungsweg ist in der DT-OS-2 708 115 die Hydrolyse von 2-Nitrobenzylbromid zum entsprechenden Alkohol und anschließende Oxidation mit verdünnter Salpetersäure beschrieben. Nachteile dieses Verfahrens sind der sehr hohe Zeitaufwand, da allein für die Hydrolyse 12 Stunden und für die Oxidation 5 Stunden Reaktionszeit benötigt werden, und das Entstehen von nitrosen Gasen, welches bei einer Salpetersäure-Oxidation unvermeidbar ist und zu besonderen Vorsichtsmaßnahmen zwingt.

Ferner wurde bekannt, aus o-Nitrobenzylbromid, Dimethylsulfoxid (DMSO) und $K_2CrO_3$ den o-Nitrobenzaldehyd herzustellen (EP-OS-3 981). Dieses Verfahren hat bei der technischen Durchführung den Nachteil, daß große Mengen anorganischer Salze bewältigt werden müssen und daß das DMSO zurückgewonnen werden muß, was bekanntlich kostenintensiv ist.

Es ist weiterhin bekannt geworden, daß o-Nitrobenzaldehyd bei der Nitrierung von Benzaldehyd bis zu einem Anteil von 20 % neben m-Nitrobenzaldehyd entsteht (vgl. W. Davey und J.R. Gwilt, J. Chem. Soc. [London] 1950, 208 ; J.W. Baker und W.G. Moffitt, J. Chem. Soc. [London] 1931, 314 ; Icke et al, Org. Synth. Coll. Vol. III, S. 644 ; O.L. Brady und S. Harris, J. Chem. Soc. [London] 1923, 484).

Bisher ist es jedoch nicht gelungen, aus diesem Nitrierungsgemisch den o-Nitrobenzaldehyd isomerenfrei, kostengünstig, technisch einfach und umweltfreundlich abzutrennen. Eine Destillation des Nitrobenzaldehydgemisches verbietet sich aus sicherheitstechnischen Gründen. Die Trennung auf « kaltem Wege », z. B. durch fraktionierte Kristallisation geeigneter Derivate, gelingt nur unvollständig (J. Chem. Soc. (London) 123, 484).

Aus Maurice M. Bursey, Tetrahedron Letters 8 (1968), S. 981-984 ; zitiert in C.A. Vol. 69 (1968) 58724e sind massenspektrometische Untersuchungen von photochemischen Reaktionen bekannt. Gemäß dem experimentellen Ausführungsbeispiel auf Seite 983, unten, wurde das Dimethylacetal des o-Nitrobenzaldehydes durch Umsetzung von reinem o-Nitrobenzaldehyd mit Methanol hergestellt. Die anschließende Destillation dient jedoch nur der Abtrennung von Lösungsmitteln und Schwefelsäure und nicht der Auftrennung eines Isomeren-Gemisches. Der Siedepunkt von 59-61 °C bei einem Druck von ca. 10 microns deutet neben der massenspektrometischen Verwendung darauf hin, daß hier nur minimale Mengen des Acetals synthetisiert wurden. Eine großtechnische Herstellung unter diesen Bedingungen, insbesondere unter so geringen Drucken, ist nur unter unverhältnismäßig hohem apparativen Aufwand durchführbar und für die Praxis wertlos.

Die vorliegende Erfindung betrifft die Herstellung von o-Nitrobenzaldehyd, dadurch gekennzeichnet, daß man ein Gemisch, welches 10 bis 30 % o-Nitrobenzaldehyd und 70 bis 90 % m-Nitrobenzaldehyd enthält, in saurem Medium die entsprechenden Acetale überführt, das o-Nitrobenzaldehydacetal destillativ bei Temperaturen von 110-180 °C abtrennt und anschließend den o-Nitrobenzaldehyd aus dem Acetal freisetzt.

Es ist ausgesprochen überraschend, daß das Acetalgemisch bei der für die Destillation erforderlichen Temperatur von 110-180 °C gefahrlos zu handhaben ist und daß das o-Nitrobenzaldehydacetal praktisch isomerenfrei und in hoher Ausbeute in einfacher Weise abdestilliert werden kann. Eine direkte Destillation des Nitrobenzaldehyd-Gemisches ist nicht möglich wegen der zu niedrig liegenden Zersetzungstemperatur des o-Nitrobenzaldehyds. Es war nicht vorhersehbar, daß durch die Verwendung der Acetale die Zersetzungstemperatur so erhöht wird, daß eine destillative Trennung problemlos durchgeführt werden kann.

Die Herstellung des Nitriergemischs erfolgt nach literaturbekannten Methoden, vorzugsweise in konzentrierter Schwefelsäure bei Temperaturen zwischen 10 und 40 °C.

Die Überführung des Nitriergemisches in die entsprechenden Acetale kann nach allen aus der Aldehyd-Chemie bekannten Methoden erfolgen (vgl. R.N. Icke et al., Org. Synth. Coll. Vol. 3, 644). Von besonderem Interesse ist die Verwendung von Dimethylacetalen die sich vorzugsweise zur destillativen Trennung eignen.

Der Verfahrensablauf kann in allgemeiner Form wie folgt beschrieben werden :

Das Aldehyd-Nitriergemisch, bestehend aus 10-30 % o-Nitrobenzaldehyd und 70-90 % m-Nitrobenzaldehyd wird in absolutem Alkohol (1-4 C-Atome, insbesondere Methanol) im sauren Medium, vorzugsweise in Gegenwart von Salzsäure oder Schwefelsäure, 60 bis 150 Stunden bei Raumtemperatur oder bei höheren Temperaturen und kürzerer Reaktionsdauer belassen und anschließend mit Alkalialkoholat, vorzugsweise Natriummethylat, neutralisiert, das Reaktionsgevon Salzsäure, 60 bis 150 Stunden bei Raumtemperatur belassen und anschließend mit Alkalialkoholat, vorzugsweise Natriummethylat, neutralisiert, das Reaktionsgemisch eingeengt und filtriert und dann das gelbliche Öl kontinuierlich oder diskontinuierlich in einer geeigneten Destillationsapparatur, vorzugsweise in einer Füllkörperkolonne, fraktioniert destilliert bei einer Badtemperatur zwischen 150 und 190 °C und einer Kopftemperatur zwischen 110 und 135 °C und anschließend das fraktioniert destillierte o- Nitrobenzaldehydacetal mit starker Säure, insbesondere mit Schwefelsäure, bei Temperaturen zwischen 10 und 60 °C in o-Nitrobenzaldehyd umgewandelt.

Beispiele

Beispiel 1

50,0 Gew.-Tle. des Aldehydgemisches aus 79 % m-Nitrobenzaldehyd und 20 % o-Nitrobenzaldehyd werden in 190 ml absolutem Methanol gelöst, mit 0,5 Vol.-Tln. konzentrierter Salzsäure versetzt und 120 Stunden bei Raumtemperatur belassen. Dann werden 0,5 Gew.-Tle Natriummethylat zugegeben, das Reaktionsgemisch eingeengt und filtriert. Man erhält 61,6 Gew.-Tle (94,5 % der The- orie) Acetalgemisch in Form eines gelblichen Öls. Die Destillation der isomeren Dimethylacetale erfolgt über eine vakuummantelverspiegelte Füllkörperkolonne mit einer Füllkörperschüttung von 700 mm und einem Innendurchmesser von 30 mm. Das Rücklaufverhältnis beträgt 100 : 2 und die Badtemperatur beträgt 169 °C, die Sumpftemperatur 158 °C und die Kopftemperatur, bei welcher die ortho-Verbindung übergeht, beträgt 125 °C. Man erhält 9,8 Gew.-Tle (80 % der Theorie) Destillat an o-Verbindung. 9,8 Gew.-Tle o-Nitrobenzaldehyd-dimethylacetal werden bei Raumtemperatur mit 50 Vol.-Tle 2 N $H_2SO_4$ 3,5 Stunden gerührt, wobei die öligen Tropfen verschwinden und ein Kristallisat entsteht. Die Suspension wird mit 50 Vol.-Tln. Methylenchlorid ausgeschüttelt, die Methylenchloridphase mit 30 Vol.-Tln. Wasser gewaschen, über Natriumsulfat getrocknet und eingeengt. Man erhält 7,5 Gew.-Tle weißgelben o-Nitrobenzaldehyd vom Schmelzpunkt 41-43 °C.

Beispiel 2

Analog Beispiel 1 werden 50 Gew.-Tle Nitrobenzaldehyd-Gemisch in 200 Vol.-Tln. absolutem Ethanol gelöst und wie in Beispiel 1 zu den Diethylacetalen umgesetzt. Man erhält 72,1 Gew.- Tle grünlich-gelbliches Öl, welches analog Beispiel 1 destilliert wird, wobei man 95 % o-Nitrobenzaldehyd-diethylacetal erhält, welches analog Beispiel 1 quantitativ zu o-Nitrobenzaldehyd vom Schmelzpunkt 41-43 °C umgesetzt wird.

**Patentansprüche**

1. Verfahren zur Herstellung von o-Nitrobenzaldehyd, dadurch gekennzeichnet, daß man ein Gemisch von nitriertem Benzaldehyd, welches 10 bis 30 % o-Nitrobenzaldehyd und 70 bis 90 % m-Nitrobenzaldehyd enthält, in saurem Medium in die entsprechenden Acetale überführt, das o- Nitrobenzaldehydacetal destillativ bei Temperaturen von 110-180 °C abtrennt und anschließend den o-Nitrobenzaldehyd aus dem Acetal freisetzt.

2. Verfahren gemäß Anspruch 1, dadurch gekennzeichnet, daß man das Nitiergemisch des Benzaldehydes in absolutem Alkohol bei Raumtemperatur acetalisiert und das Acetalgemisch bei Kopftemperaturen zwischen 110 und 135 °C fraktioniert destilliert und anschließend nach üblichen Methoden die Acetalgruppe abspaltet.

3. Verfahren gemäß Anspruch 1, dadurch gekennzeichnet, daß man die Acetalisierung des Nitriergemisches in Gegenwart von Salzsäure durchführt.

4. Verfahren gemäß Anspruch 1, dadurch gekennzeichnet, daß man die Abspaltung der Acetalgruppe bei Temperaturen zwischen 10 und 60 °C in Gegenwart von Schwefelsäure durchführt.

5. Verfahren gemäß Anspruch 1 und 2, dadurch gekennzeichnet, daß man die fraktionierte Destillation an einer Füllkörperkolonne mit Badtemperaturen von 150 bis 190 °C durchführt.

6. Verfahren gemäß Ansprüchen 1, 2 und 5, dadurch gekennzeichnet, daß man Dimethyl- oder Diethylacetale einsetzt.

**Claims**

1. Process for the preparation of o-nitrobenzaldehyde, characterised in that a mixture of nitrated benzaldehyde, which contains 10 to 30 % of o- nitrobenzaldehyde and 70 to 90 % of m-nitrobenzaldehyde, is converted, in an acid medium, into the corresponding acetals, the o-nitrobenzaldehyde acetal is separated off by distillation at temperatures of 110-180 °C and then the o-nitrobenzaldehyde is liberated from the acetal.

2. Process according to Claim 1, characterised in that the nitration mixture of the benzaldehyde is acetalated in absolute alcohol at room temperature and the acetal mixture is fractionally distilled at overhead temperatures of between 110 and 135 °C and then the acetal group is split off by customary methods.

3. Process according to Claim 1, characterised in that the acetalation of the nitration mixture is carried out in the presence of hydrochloric acid.

4. Process according to Claim 1, characterised in that the splitting off of the acetal group is carried out in the presence of sulphuric acid at temperatures of between 10 and 60 °C.

5. Process according to Claim 1 and 2, characterised in that the fractional distillation is carried out in a packed column with bath temperatures of 150 to 190 °C.

6. Process according to Claims 1, 2 and 5, characterised in that dimethyl or diethyl acetals are used.

**Revendications**

1. Procédé de production de o-nitrobenzaldéhyde, caractérisé en ce qu'on transforme un mélange de benzaldéhyde nitré, qui contient 10 à 30 % de o-nitrobenzaldéhyde et 70 à 90 % de m-nitrobenzaldéhyde, en milieu acide en les acétals correspondants, on sépare le o-nitrobenzaldéhyde-acétal par distillation à des températures de 110 à 180 °C, puis on libère le o-nitrobenzaldéhyde de l'acétal.

2. Procédé suivant la revendication 1, caractérisé en ce qu'on acétalise le mélange nitré de benzaldéhyde dans de l'alcool absolu à la température ambiante et on soumet le mélange d'acétals à une distillation fractionnée à des températures de tête comprises entre 110 et 135 °C, puis on scinde le groupe acétal par des opérations classiques.

3. Procédé suivant la revendication 1, caractérisé en ce qu'on effectue la transformation en acétals du mélange nitré en présence d'acide chlorhydrique.

4. Procédé suivant la revendication 1, caractérisé en ce que la scission du groupe acétal est effectuée à des températures comprises entre 10 et 60 °C en présence d'acide sulfurique.

5. Procédé suivant les revendications 1 et 2, caractérisé en ce qu'on conduit la distillation fractionnée sur une colonne à garniture, à des températures du bain de 150 à 190 °C.

6. Procédé suivant les revendications 1, 2 et 5, caractérisé en ce qu'on utilise des diméthylacétals ou des diéthylacétals.